# FASCICULE DE BREVET EUROPEEN

(11) **EP 1 058 849 B1**
(45) Date de publication et mention de la délivrance du brevet: **31.03.2004**
(21) Numéro de dépôt: 99904920.8
(22) Date de dépôt: 22.02.1999
(51) Int. Cl.: G01N 33/50

(54) **BIOCAPTEUR ENVIRONNEMENTAL DE METROLOGIE D'AEROCONTAMINANTS**
BIOSENSOR ZUR MESSUNG VON LUFTVERUNREINIGUNGEN
ENVIRONMENTAL BIOSENSOR FOR MEASURING AIR CONTAMINANTS

(30) Priorité: 27.02.1998 FR 9802376
(43) Date de publication de la demande: 13.12.2000
(73) Titulaire: LECES, 57280 Maizières-lès-Metz (FR)
(72) Inventeur: FALLA-ANGEL, Jairo, Alberto, F-57000 Metz (FR); LAVAL-GILLY, Philippe, F-57100 Thionville (FR); HENRYON, Michel, F-54920 Morfontaine (FR)
(74) Mandataire: Ventavoli, Roger
(86) Numéro de dépôt international: PCT/FR1999/000387
(87) Numéro de publication internationale: WO 1999/044061

(56) Documents cités:
- WO-A-90/04645
- WO-A-97/13871
- DATABASE WPI Section Ch, Week 9747 Derwent Publications Ltd., London, GB; Class B04, AN 97-511202 XP002081726 & RU 2 079 130 C (ST PETERSBURG TRAUMATOLOGY ORTHOPAEDICS), 10 mai 1997
- C. ARANYI ET AL: "Evaluation of potential inhalation hazard of particulate silicious compounds by in vitro rabbit alveolar macrophage tests - application to industrial particulates containing hazardous impurities." AMERICAN SOCIETY FOR TESTING AND MATERIALS SPEC. TECH. PUBL., HEALTH EFFECTS OF SYNTHETIC SILICA PARTICULATES, vol. 732, 1981, pages 48-61, XP002081724
- R. HOLT: "Effects of air pollution on the upper aerodigestive tract." OTOLARYNGOLOGY - HEAD AND NECK SURGERY, vol. 114, no. 2, février 1996, pages 201-204, XP002081725

## Description

La présente invention a trait à la métrologie d'aérocontaminants par mesure de grandeurs caractéristiques de variations comportementales des monocytes et macrophages de défense de l'appareil respiratoire humain en présence de tels aérocontaminants.

On rappelle que les monocytes et macrophages dont il est question ici sont des cellules phagocytaires de défense immunitaire présentes en mobilité libre sur l'épithélium alvéolaire, à l'interface air-liquide des alvéoles pulmonaires, et dont la mission est de débarrasser en permanence la muqueuse respiratoire des particules, micro-organismes, ou autres contaminants atmosphériques ingérés par inhalation et provenant de la pollution de l'air.

La quantification de l'agressivité sur l'homme, en termes de nuisance à la santé publique, des aérocontaminants de l'ère moderne est un devenue un préalable nécessaire aux prises de mesures attendues de lutte contre la pollution atmosphérique grandissante. Parmi les aérocontaminants, ceux qui intéressent ici sont d'abord les polluants particulaires les plus ténus, dont la taille infime (inférieure à 3 µm environ) leur permet de franchir les barrages de défense filtrants de la partie supérieure de l'appareil respiratoire (fosses nasales, bouche, rhino-pharynx) et de la partie moyenne (dispositif muco-cilliaire des bronches), pour atteindre le poumon alvéolaire profond où ils ne pourront être évacués à ce stade ultime que par les mécanismes phagocytaires de l'immunité cellulaire non spécifique mis en oeuvre par les monocytes et macrophages alvéolaires. On citera sans exhaustivité les poussières de silice, d'amiante, mais aussi celles d'oxydes de métaux lourds comme le plomb, le cadmium ou le sélénium, qui de surcroît ont tous une action dépressive sur le nombre de macrophages pulmonaires ou sur l'efficacité de leur action de défense.

C'est précisément cette action dépressive qui est prise en compte par l'invention, car, non seulement ces micro-particules sont nocives en elles-mêmes, mais elles obèrent en outre la capacité des macrophages à assumer leur mission d'élimination des agents pathogènes habituels véhiculables par l'atmosphère, que sont les micro-organismes, staphylocoques, streptocoques, aspergillus, spores de microchampignons, etc....

Il convient de rappeler ici qu'existent des aérocontaminants du poumon profond autres que les poussières et qui sont tout autant inhibiteurs de la phagocytose macrophagique et monocytaire, à savoir les polluants gazeux, notamment d'origine urbaine ou industrielle. Il s'agit en particulier des hydrocarbures résultant de la mauvaise combustion des énergies fossiles, ou dérivées comme les carburants des véhicules de transport, ou les solvants de l'industrie chimique et les gaz oxydants. On citera par exemple les HAP (hydrocarbures aromatiques polycycliques) dont le DMBA (7-12 diméthyl-benzène-anthracène), ou le benzène lui-même dont l'action limitante de la phagocytose monocytaire et macrophagique a pu être démontrée, de même que celle des oxydes d'azote (NOₓ), ou de l'ozone.

Jusqu'à présent, les méthodes d'évaluation de la pollution atmosphérique s'attachent à prendre en compte les propriétés physico-chimiques des molécules mises en cause. Par ailleurs, l'action de certains polluants atmosphériques sur la chimie de la cellule humaine ou sur sa durée de vie, est étudiée de son côte. On peut citer à cet égard les travaux de l'équipe de C. VOISIN publiés à la fin des années 70, notamment dans le Bulletin européen de physiologie respiratoire (p. 137 à 144, ou p. 69 à 82 du n° 13 de 1977; p. 416 à 419 du n° 54 de 1979). Toutefois, il n'existe toujours pas à ce jour, à la connaissance du Demandeur, d'instrument de mesure permettant de faire le lien direct entre l'évaluation de la pollution atmosphérique et l'impact quantifié d'un polluant identifié sur l'organisme humain.

La présente invention à précisément pour but de proposer un tel appareil, qui soit sensible, suffisamment rustique et automatisé pour fonctionner tant au laboratoire qu'en routine en site naturel, et fournir de façon fiable l'information voulue quasi-instantanément.

A cet effet, l'invention a pour objet un biocapteur environnemental de métrologie des aérocontaminants caractérisé en ce qu'il comprend :
- une source de culture de cellules de défense immunitaire de l'organisme humain (animal en général), tels que des monocytes et macrophages alvéolaires de l'appareil respiratoire;
- une ligne de prélèvement d'échantillons d'analyse de l'atmosphère contaminée sous forme d'un flux gazeux à débit contrôlé,
- une cellule d'exposition définissant une enceinte destinée à être balayée par un courant gazeux et dont le fond, en matériau transparent à la lumière, est conçu pour être tapissé par une nappe liquide renouvelable et mise au contact dudit courant gazeux, cette cellule étant pourvue, d'une part, d'une entrée/sortie pour l'établissement du courant gazeux de balayage de l'enceinte, l'entrée étant reliée à la sonde de prélèvement atmosphérique afin que ledit courant gazeux soit l'échantillon atmosphérique à analyser, et, d'autre part, une entrée/sortie pour l'établissement de la nappe liquide sur le fond, l'entrée étant reliée à ladite source de culture afin que cette dernière forme ladite nappe;
- des moyens de rinçage du fond de la cellule d'exposition;
- et un système d'observation du fond de la cellule comprenant une optique grossissante, tel qu'un microscope, associée à une caméra vidéo, des moyens d'acquisition des signaux d'images-sources produits par la caméra, des moyens de traitement de ces images-sources pour élaborer des images-traitées aptes à révéler des modifications du comportement des macrophages sous l'influence de l'échantillon atmosphérique gazeux au contact de la nappe liquide contenant lesdits macrophages, et des moyens de visualisation desdites images traitées.

Conformément à une réalisation préférée, les moyens de traitement des images-sources fournies par la caméra sont des moyens aptes à révéler les modifications de la mobilité, en direction d'une arrivée d'agents chimio-attractifs, des monocytes et macrophages sous influence de l'échantillon gazeux atmosphérique à analyser, et le biocapteur de l'invention comporte en outre, à cet effet, une source d'agents chimio-attractifs des macrophages, ainsi que, reliée à cette source, une entrée dans la cellule d'exposition pour l'arrivée desdits agents, placée de préférence en un endroit éloigné de celui de l'entrée des macrophages.

Comme on le comprend, l'invention consiste donc, dans ses caractéristiques essentielles, en une association entre microscopie de laboratoire et analyse électronique d'images afin de mesurer les variations de grandeurs représentatives du comportement des monocytes et macrophages, comme leur mobilité en termes de vitesse de déplacement en direction d'une cible par exemple (agents chimio-attractifs), ou leurs changements de couleur, ou leurs modifications morphologiques lorsqu'ils sont placés en situation de devoir réagir à la présence d'agents indésirables externes véhiculés par l'atmosphère.

Dans ce cadre, l'invention a également pour objet un procédé d'utilisation du biocapteur précédemment défini dans sa version équipée d'une source d'agents chimio-attractifs des monocytes et macrophages délivrant ces agents dans la cellule d'exposition, procédé caractérisé en ce que, dans le but de quantifier l'influence directe de contaminants atmosphériques sur le comportement des monocytes et macrophages alvéolaires, on utilise ledit capteur pour exprimer les changements de vitesse avec laquelle ceux-ci se déplacent en direction de la cible formée par ces agents chimio-attractifs lorsque ces monocytes et macrophages se trouvent dans la nappe sur le fond de la cellule d'inspection au contact de l'échantillon d'atmosphère contaminée placée au dessus.

L'invention sera bien comprise et d'autres aspects et avantages apparaîtront plus clairement au vu de la description qui suit d'un exemple de réalisation du bio-capteur en référence aux planches de dessins annexées sur lesquelles:
- la figure 1 est un schéma d'ensemble représentatif du bio-capteur de l'invention dans une forme complète de réalisation et de son mode de fonctionnement;
- la figure 2 est une vue en coupe verticale de la cellule d'exposition selon une forme préférée de réalisation;
- la figure 3 illustre le principe d'une méthode d'analyse de la nocivité d'aéro-contaminants sur l'homme basée sur la mobilité des monocytes et macrophages alvéolaires en direction d'une cible;
- la figure 4 est une représentation graphique de résultats bruts obtenus de quantification de cette nocivité à l'aide d'un indicateur de mobilité des monocytes et macrophages constitué par la mesure de leur trace sur le fond, exprimée comme la surface balayée sur ce fond en fonction du temps par la population de cellules macrophagiques et monocytaire s simultanément présente dans la cellule d'observation.

Par souci de clarté de l'exposé, on désignera par la suite l'ensemble "monocytes et macrophages" sous le vocable simplifié de "macrophages"

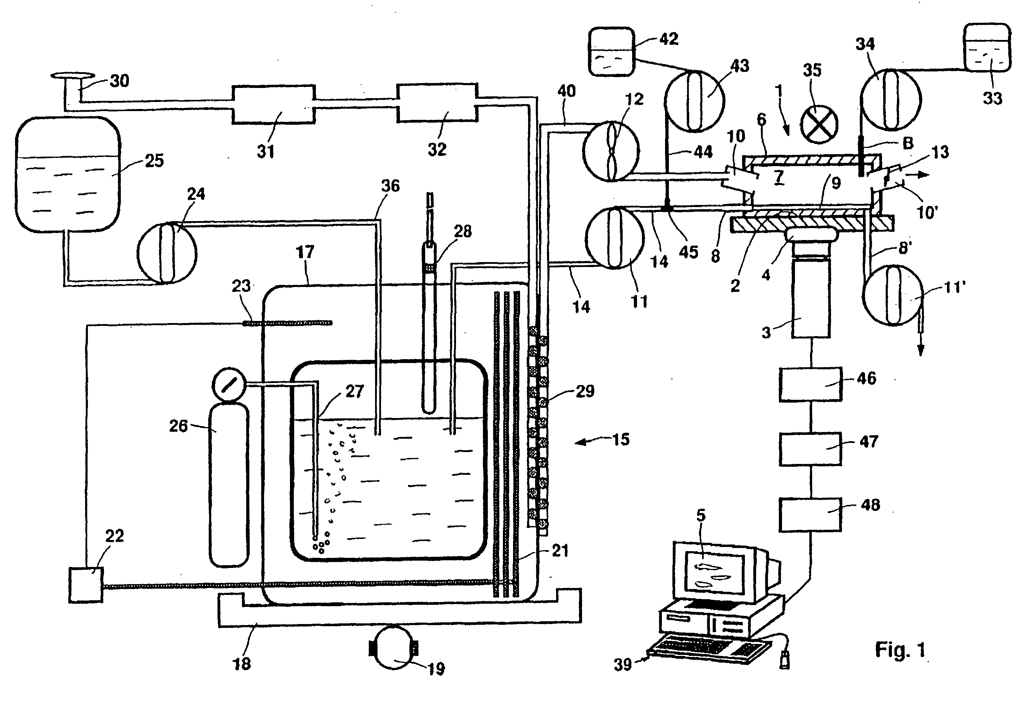

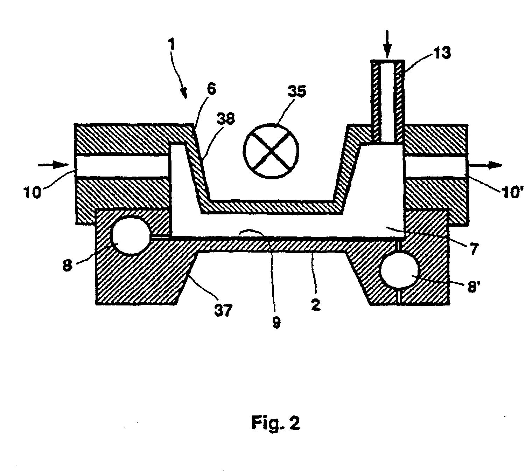

En se reportant d'abord sur les figures 1 et 2, on voit que le bio-capteur comprend une cellule d'exposition 1 dont le fond 2, en matériau translucide, tel que du polypropylène , est placé dans le champ d'une caméra vidéo 3 couplée à un microscope 4 faisant office d'objectif. La sortie de la caméra est reliée à une chaîne de traitement aboutissant à un moniteur de visualisation 5 affichant une image quantitativement représentative du déroulement des événements intervenus sur le fond 2 de la cellule 1 pendant la période d'observation.

Le fond 2, profilé en cuvette plate, est surmonté par un capot jointif 6 de manière à définir ensemble une cellule 1 étanche. L'enceinte intérieure 7 de la cellule 1 communique avec l'extérieur au moyen d'un certain nombre d'ouvertures pour le passage de fluides que l'on énumère à présent:
- un premier couple d'Entrée/Sortie 8, 8', conformées de préférence en fentes horizontales, débouchant au voisinage immédiat du fond 2, et destinées à permettre de le tapisser par une nappe liquide 9, renouvelable quand on le désire grâce à des pompes rotatives 11 et 11' commandées respectivement pour l'introduction du liquide dans l'enceinte 7, puis pour son évacuation;
- un second couple d'Entrée/Sortie 10, 10' pour le balayage de l'enceinte 7 par un courant gazeux mû par un ventilateur soufflant 12 à débit réglable. La pression du gaz dans l'enceinte 7 est réglé à l'aide d'un organe de perte de charge variable 13 (une vantelle par exemple) monté dans le passage de sortie 10';
- une Entrée supplémentaire 13 pour déposer sur le fond en quantité ajustée une substance liquide, solide ou pâteuse, en un endroit placé de préférence à l'opposé de l'arrivée 8 pour des raisons qui apparaîtront par la suite;

L'Entrée 8 est reliée, par une conduite 14 pourvue de la pompe 11, à un réacteur biologique 15 composé d'une cuve 16 logée dans une enceinte thermostatée 17, le tout étant posé sur un socle 18 animé par un moteur 19 pour assurer un brassage d'homogénéisation du contenu de la cuve. La régulation en température est menée à l'aide d'éléments radiants 21 pilotés par un régulateur 22 relié à un thermomètre 23 repérant la température à l'intérieur de l'enceinte thermique 17.

La cuve 16 est remplie par un liquide 20 constitué, dans l'exemple considéré, par une culture entretenue de macrophages que l'on trouve dans les alvéoles pulmonaires de l'homme. La cuve 16 est de préférence gérée à niveau de liquide 20 constant. Aussi, les sorties de culture par la conduite 14 sont compensées tant que de besoin par un apport « frais » provenant d'un réservoir d'appoint 25 à l'aide d'une pompe 24 associée à une tubulure d'arrivée 36. Le réacteur 15 est également pourvu d'une source de CO₂ sous pression 26 délivrant, par une canule plongeante 27, un débit de barbotage dans la culture 20 y assurant un degré d'oxydation adéquat à la survie des macrophages. Une sonde de prélèvement 28 est avantageusement prévue pour pouvoir procéder de temps à autre à l'analyse du milieu de culture 20.

Le capteur de l'invention est encore doté de moyens de rinçage du fond de la cellule. Un tel rinçage s'avère systématiquement souhaitable après l'évacuation d'une nappe « usagée » 9 afin que l'installation d'une nappe nouvelle se fasse sur un fond 2 bien propre, non susceptible de fausser ou encombrer les mesures suivantes. Comme on le voit sur la figure 1, ces moyens de rinçage peuvent être simplement constitués d'un réservoir 42 contenant une solution de lavage adéquate et d'une conduite 44 pourvue d'une pompe 43 et reliant ce réservoir à la conduite 14 d'alimentation de l'entrée 8 par un piquage 45 placé en aval de la pompe 11.

On observe sur la figure 1, que les éléments radiants 21 servent également à chauffer un serpentin 29 intérieurement parcouru par un flux d'air aspiré depuis une prise d'air atmosphérique sous abri 30 au moyen du ventilateur 12 placé entre le serpentin est l'Entrée 10 de la cellule d'observation.

Le flux régulier et contrôlé en débit d'air aspiré par la bouche 30 constitue l'échantillon d'air atmosphérique à analyser par le bio-capteur selon l'invention.

Dès son captage par la bouche extérieure 30, l'air aspiré est grossièrement filtré en 31 pour retenir d'éventuels insectes et assurer une protection de la ligne de prélèvement à l'égard d'actes malveillants. L'air est ensuite asséché par passage dans un condenseur 32 avant d'être réchauffé à la température d'analyse (entre 35 et 40°C) dans le serpentin 29 et insufflé dans l'enceinte 7 de la cellule 1 par la fente d'entrée 8 à l'aide du ventilateur 12. L'ensemble constitué par les éléments qui viennent d'être énoncés et chaînés entre eux dans l'ordre décrit, constitue la "ligne de prélèvement des échantillons atmosphériques contaminés" à analyser par mesure de leur influence sur la mobilité des macrophages présents dans la nappe 9 au sein de la cellule d'exposition 1.

A cet effet, la conduite d'arrivée 13 est reliée à une source 33 d'agents chimio-attractifs des macrophages par l'intermédiaire d'une pompe dosimétrique 34 permettant de délivrer en un endroit localisé sur le fond 2 une quantité mesurée desdits agents servant de cible reconnaissable par les macrophages. De préférence, comme déjà signalé, cet endroit sera éloigné de celui de l'arrivée 8 du liquide contenant les macrophages afin de disposer ainsi d'un temps suffisant pour observer la mobilité de ces derniers attirés par la cible avant qu'il ne s'immobilisent contre elle. Les agents chimiques pouvant servir de cible sont bien connus, comme par exemple les lipopolysacharides (ou LPS), ou les n-formyl Méthionine Leucine Phénilalanine (ou fMLP).

Comme on le voit, un éclairage 35 est avantageusement prévu au niveau de la cellule 1 afin d'améliorer par contraste lumineux les conditions d'observation de la nappe 9 par la caméra 3 au travers du fond 2. Cet éclairement peut être direct, comme le montre la figure 1, auquel cas le capot 6 doit également être en matériau translucide et le microscope 4 pourra être doté d'un filtre d'entrée anti-éblouissement. L'éclairement peut également être de type « indirect », l'organe d'éclairage étant alors placé du même coté que la caméra par rapport au fond de la cellule 1.

Quoiqu'il en soit, on aura toujours avantage à amincir le fond 2 à l'endroit d'implantation du microscope 4, comme le montre l'évidement 37 de la figure 2. En l'espèce, il est vrai que le fond 2 a été rendu particulièrement massif, puisqu'il accueille des Entrée-sortie 8, 8' sous forme de chambres à fentes intérieures, ainsi que des résistances chauffantes non représentées pour maintenir au besoin la nappe 9 à une température constante, de préférence égale ou voisine de 37°C. De même, on voit que le capot 6 présente en renfoncement central 38 destiner à loger l'organe d'éclairage 35 afin de le rapprocher au maximum de la nappe 9 à éclairer.

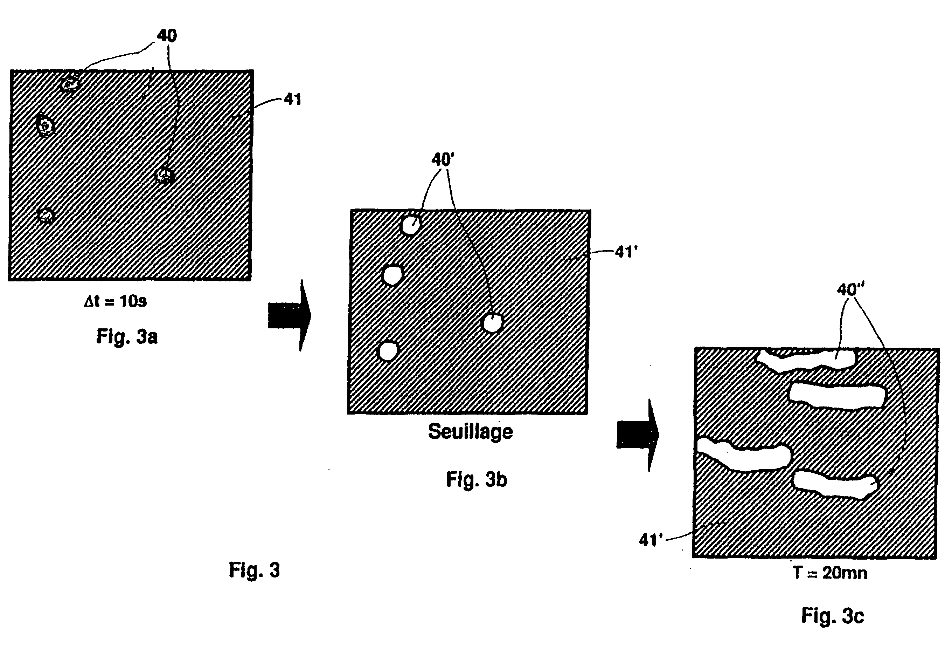

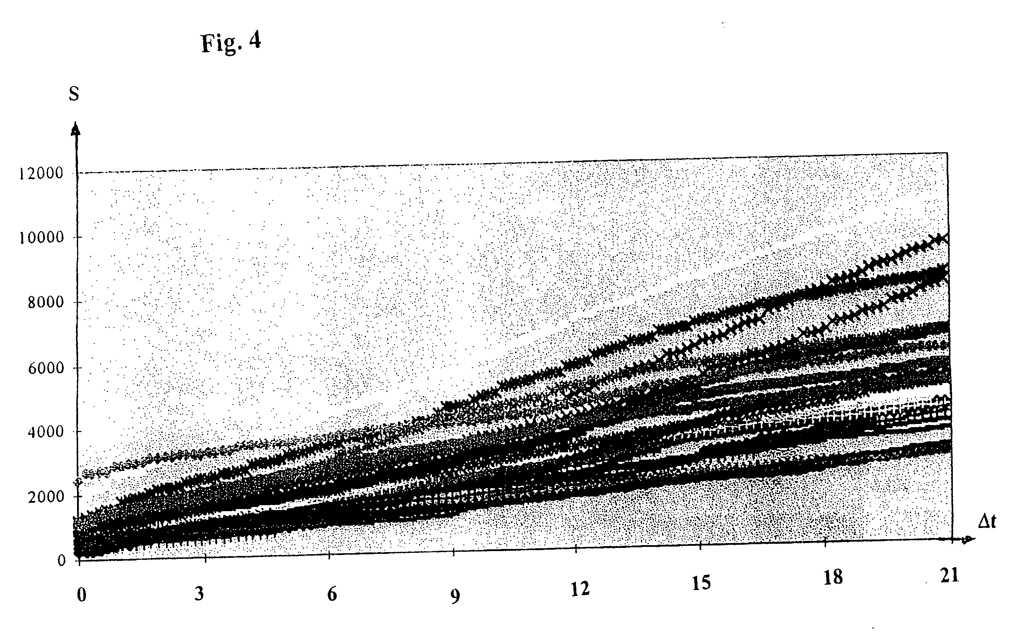

La portion de nappe 9 présente dans le champ du microscope 4 (donc filmée en permanence par la caméra vidéo 3) se traduit à la sortie de la caméra 3 par une image-source devant être traitée dans une chaîne de traitements électroniques d'images qui se termine par la restitution sur le moniteur 5 d'un micro-ordinateur 39 d'une image-objet finale représentative de la mobilité relative des macrophages selon qu'ils sont, ou non, mis au contact dans la cellule 1 avec le flux d'air atmosphérique contaminé à analyser. Cette ligne de traitement d'images est composée, dans l'ordre chronologique du traitement, par
a) une numérisation 46 de l'image-source, qui s'opère pendant une quinzaine de minutes avec une période de saisie de 10 secondes;
b) suivie d'une étape de prétraitement 47, laquelle permet d'identifier les cellules macrophages mortes et les particules immobiles, et les isoler du reste pour clarifier l'image-objet finale. A cette fin, les 20 premières images environ sont traitées et comparées entre-elles pour la constitution d'un masque;
c) puis une étape de traitement proprement dite 48, dans laquelle sont extraits les paramètres représentatifs de la métrologie souhaitée sur les macrophages sous influence des aérocontaminants, comme la mobilité (vitesse moyenne de déplacement et orientation), leur taille, leur couleur, etc.... Dans cette étape, les images numérisées en a) subissent un traitement morphologique des gris et un seuillage afin de construire la silhouette des macrophages 40 sous forme de pastilles blanches 40' (fig. 3b) se détanchant du fond sombre 41'. Une opération de comparaison avec le masque obtenu en b) permet d'éliminer de l'image les cellules mortes et particules immobiles pour ne retenir que les macrophages vivants, et une compilation de toutes les images-sources conduit alors à une image-objet finale 3c faisant apparaître chaque macrophage selon sa trace qu'il génère au cours de son déplacement pendant la durée d'observation considérée. L'orientation et la longueur de chaque trace est directement représentative de la mobilité des macrophages sous influence. Une mise en forme mathématique des différentes images traitées permet ainsi la quantification des paramètres suivis et afficher par exemple sur l'écran 5 les courbes de mobilité de la figure 4;
d) un enregistrement des données dans la mémoire du l'ordinateur 39 afin d'assurer la tracabilité des analyses, ce qui est un préalable nécessaire à l'affichage précité des résultats sur le moniteur 5

Le principe de fonctionnement du capteur de l'invention, tel que représenté à la figure 1 dans son application "mobilité des macrophages ", peut être résumé de la façon suivante:
1) Sur commande manuelle ou automatique, la pompe 11 alimente l'arrivée 8 avec une quantité de milieu de culture 20 ajustée pour former sur le fond 2 une nappe 9 d'épaisseur régulière de quelques dixièmes de mm seulement. La sortie 8' est alors close et la pompe de vidange 11' inactive. Le milieu de culture 20 est géré dans le réacteur 15 en rapport avec le réservoir d'appoint 25 de manière que la nappe 9 contienne des macrophages en nombre ni trop bas, ni trop élevé, par exemple une dizaine pour une surface de nappe 9 de l'ordre de 0,1 dm² environ.
2) La nappe 9 une fois installée, on déverse par la conduite 13 quelques gouttes d'agents chimio-attractif contenu dans la réserve 33 et qui tombent par gravité sur la nappe 9 au voisinage de la fente de sortie 8'.
3) On peut alors procéder à l'acquisition des images du fond par la caméra 3 et à leur traitement selon les séquences 46, 47 et 48 décrites précédemment. Une phase de mise au point préliminaire consiste à repérer dans la nappe 9 les macrophages morts (il y en a quasi-inévitablement) afin de retenir pour la restitution des résultats que les vivants. A partir d'une valeur limite prédéterminée librement choisie exprimant par exemple la surface minimale que doit balayer un macrophage sur le fond pendant cette période pour pouvoir être qualifié de vivant, on fait alors le tri entre les macrophages vivants dont la surface balayée associée est supérieure à cette valeur limite, et les autres. Par la technique du "masquage", ces derniers sont alors éliminés pour ne pas encombrer inutilement l'image finale restituée par le moniteur 5. Cette opération permet également d'éliminer de l'image des particules inertes éventuellement présentes dans le milieu de culture 20.
4) On peut par ailleurs avantageusement améliorer le contraste de l'image à restituer sur l'écran 5 par le traitement 47 de seuillage des gris de l'image source captée par la caméra 3 de manière, par exemple, à faire apparaître les macrophages vivants en clair sur fond sombre. Cette opération de contrastage est illustrée par la figure 3, où l'on voit en 3 a l'image source issue d'une période d'observation d'environ 10 sec extraite par l'échantillonneur 46. et sur laquelle, après application du "masque", on peut distinguer, sur fond grisâtre 41 représentant le milieu de culture des macrophages, des objets circulaires 40, de tailles analogues, eux-mêmes de couleur plutôt grise et qui en l'espèce sont tous des macrophages alvéolaires vivants. Après seuillage des gris, on obtient l'image 3b où les macrophages apparaissent selon des pastilles blanches 40' qui se détachent cette fois très nettement du reste 41' passé au très sombre. Ce sont ces macrophages 40' qui vont ensuite se diriger vers leur cible chimio-attractive en formant au cours du temps une trace blanche révèlée par le traitement 48. Ces traces sont bien visibles sur la figure 3c résultant de l'évolution de l'image 3b sur une vingtaine de minutes de temps d'observation de la caméra. On notera que le temps d'observation nécessaire au seuillage (une dizaine de sec.) est suffisamment court, eu égard la mobilité des macrophages, pour pouvoir effectuer ce traitement de contrastage alors que l'agent attractif est déjà injecté dans la cellule 1.

L'image-objet finale restituée sur l'écran 5 peut être analogue à celle de la figure 3a. On comparera alors entre elles les traces 40' des macrophages obtenues sur deux images-objet à durées d'exposition déterminées, voire identiques, mais réalisées sans et en présence d'un flux d'air aérocontaminé balayant la cellule d'exposition 1 pour en déduire, en fonction de la nature des contaminants, la diminution de la mobilité des macrophages placés sous leur influence et caractériser ainsi leur niveau de nocivité sur l'organisme humain. Une telle caractérisation sera aisément quantifiable si on a prévu d'établir au préalable un étalonnage du capteur à l'aide d'échantillons de référence d'air contaminé dont l'analyse est rigoureusement connue et stable, et dont on mesure l'influence de chacun sur la mobilité des macrophages pour construire des abaques de lecture directe de nocivité à partir des mesures de mobilité données par le capteur.

Une fois l'opération terminée, la pompe de vidange 11' est activée pour évacuer du fond 2 la nappe 9 avec la dose d'agents chimio-attracteurs qui lui a été ajoutée. On procède ensuite à un rinçage du fond 2 avec de la solution de lavage biologique apportée depuis le réservoir 42 par la conduite 44. Dans cette phase, les deux pompes 12 et 11' sont simultanément actives pour assurer un lavage vigoureux du fond de la cellule 1, avant de procéder à l'installation d'une nouvelle nappe 9 en vue d'une nouvelle analyse.

Bien entendu, le capteur de l'invention peut également servir, non seulement en alerte si des seuils critiques en ralentissement de mobilité sont dépassés, mais également en tant qu'outil de recherche pour étudier l'influence de polluants nouveaux ou encore incomplètement identifiés. La figure 4 montre une image-objet restituée sur le moniteur 5 qui illustre bien ce que l'on peut attendre du capteur à ce sujet. Le temps d'observation Δt (en mn) est en abscisse, et l'ordonnée donne, exprimée en nombre de pixels² d'écran 5, la surface de la traînée des macrophages. La figure 4 accueille une trentaine de courbes, chacune étant représentative d'un échantillon d'analyse donné se différenciant des autres par un aérocontaminant contenu différent soit en nature, soit en concentration. Comme on le voit, chaque courbe présente en fait un tracé quasi-linéaire avec une pente moyenne correspondant à un accroissement de la trace d'approximativement 2000 pixels² en 10 mn en moyenne. Un échantillon cependant échappe à ce constat et se distingue également du reste par son ordonnée à l'origine importante (plus de 2000 pixels²) traduisant le fait qu' une quantité sensiblement plus importante de cellules macrophages a été analysée pour cet échantillon.

Il va de soi que l'invention ne se limite pas aux exemples décrits de réalisation et d'utilisation du capteur, mais s'étend à de multiples variantes ou équivalents dans la mesure où demeure respectée la définition du capteur donnée par les revendications jointes.

En particulier, d'autres caractéristiques comportementales des macrophages que leur mobilité peuvent être quantifiées en réponse à la présence d'aérocontaminants. En début de mémoire, on a cité à cet égard les variations de couleur, mais on peut rajouter des modifications morphologiques ou physiologiques.

## Revendications

1. Biocapteur environnemental de métrologie des aérocontaminants, **caractérisé en ce qu'**il comprend :
- une source de culture (15) de cellules de défense immunitaire de l'organisme humain, tels que des monocytes et macrophages alvéolaires, sensibles à ces aérocontaminants ;
- une sonde de prélèvement (30,12) d'échantillons d'analyse représentatifs de l'atmosphère contaminée, et apte à délivrer les échantillons sous forme d'un flux gazeux à débit contrôlé,
- une cellule d'exposition (1) définissant une enceinte étanche (7) destinée à être balayée par un courant gazeux et dont le fond (2), en matériau translucide, est conçu pour être tapissé par une nappe liquide (9) au contact dudit courant gazeux, ladite cellule (1) étant pourvue, d'une part, d'une entrée/sortie (10, 10') pour l'établissement du courant gazeux de balayage de l'enceinte (7), l'entrée (10) étant reliée à la sonde de prélèvement atmosphérique (30,12) et d'autre part, d'une entrée/sortie (8, 8') pour l'établissement de la nappe liquide (9) sur le fond (2), l'entrée (8) étant reliée à ladite source (15) de culture (20) afin que cette dernière forme ladite nappe ;
- des moyens de rinçage (42, 45) du fond (2) de ladite cellule d'exposition;
- et un système d'inspection du fond (2) de la cellule d'exposition comprenant une optique grossissante (4) associée à une caméra (3), des moyens d'acquisition des signaux d'images-sources produits par la caméra, des moyens (46, 47, 48, 39) de traitement desdits signaux pour élaborer des images-traitées aptes à révéler des modifications comportementales des cellules de défense immunitaire, et des moyens (5) de visualisation desdites images traitées.

2. Biocapteur selon la revendication 1, **caractérisé en ce que** ladite source (15) contient une culture de monocytes et macrophages alvéolaires de l'appareil respiratoire humain.

3. Biocapteur selon la revendication 1, **caractérisé en ce qu'**il comporte en outre une source d'agents chimio-attractifs (33) des cellules de défense, ainsi que, reliée à cette source, une entrée (13) dans la cellule d'exposition (1) pour l'arrivée desdits agents chimio-attractifs, et **en ce que** les moyens de traitement (46,47) des images-sources fournies par la caméra (3) sont des moyens aptes à révéler les modifications de la mobilité, en direction du lieud'arrivée des agents chimio-attractifs sur le fond (2) des cellules de défense sous influence de l'échantillon gazeux atmosphérique à analyser.

4. Biocapteur selon la revendication 1, **caractérisé en ce qu'**il comprend des moyens d'éclairement (35) du fond (2) de la cellule d'observation (1).

5. Biocapteur selon la revendication 1, **caractérisé en ce que** les moyens de rinçage du fond (2) de la cellule d'exposition (1) sont reliés à l'entrée (8) de la nappe liquide (9).

## Claims

1. An environmental biosensor for the metrology of aerocontaminants, **characterized in that** it comprises:
- a source of culture (15) of cells for the immune defence of the human body, such as alveolar monocytes and macrophages, sensitive to these aerocontaminants;
- a probe for sampling (30, 12) analytical samples representative of the contaminated atmosphere, and capable of delivering the samples in the form of a gaseous stream with controlled flow rate,
- an exposure cell (1) defining a leakproof chamber (7) intended to be swept by a gaseous stream and, whose bottom (2) , made of translucent material, is designed to be covered with a liquid layer (9) in contact with said gaseous stream, said cell (1) being provided, on the one hand, with an inlet/outlet (10, 10') for establishing the gaseous stream for sweeping the chamber (7), the inlet (10) being connected to the probe for atmospheric sampling (30, 12) and, on the other hand, an inlet/outlet (8, 8') for establishing the liquid layer (9) on the bottom (2) , the inlet (8) being connected to said source (15) of culture (20) so that the latter forms said layer;
- means for rinsing (42, 45) the bottom (2) of said exposure cell;
- and a system for inspecting the bottom (2) of the exposure cell comprising a magnifying optic (4) combined with a camera (3), means for acquiring source-image signals produced by the camera, means (46, 47, 48, 39) for processing said signals in order to produce processed images capable of revealing behavioural modifications of the immune defence cells, and means (5) for visualizing said processed images.

2. The biosensor according to claim 1, **characterized in that** said source (15) contains a culture of alveolar monocytes and macrophages of the human respiratory apparatus.

3. The biosensor according to claim 1, **characterized in that** it comprises, in addition, a source of chemoattractive agents (33) for the defence cells, as well as, connected to this source, an inlet (13) in the exposure cell (1) for the admission of said chemoattractive agents, and **in that** the means for processing (46, 47) the source images provided by the camera (3) are means capable of revealing modifications in mobility, in the direction of the site of admission of the chemoattractive agents on the bottom (2) for the defence cells under the influence of the gaseous atmospheric sample to be analysed.

4. The biosensor according to claim 1, **characterized in that** it comprises means of illuminating (35) the bottom (2) of the observation cell (1).

5. The biosensor according to claim 1, **characterized in that** the means for rinsing the bottom (2) of the exposure cell (1) are connected to the inlet (8) of the liquid layer (9).

## Patentansprüche

1. Umweltbiosensor zur Messung von Luftverunreinigungen, **dadurch gekennzeichnet, dass** er beinhaltet:
- eine Quelle (15) zur Kultur von Immunabwehrzellen des menschlichen Organismus, wie z. B. alveoläre Monozyten und Makrophagen, die gegen diese Luftverunreinigungen empfindlich sind;
- eine Sonde (30, 12) zur Entnahme von Analysenproben, die für die verunreinigte Atmosphäre repräsentativ sind, und zur Lieferung von Proben in Form eines mengenkontrollierten Gasflusses;
- eine Expositionszelle (1), die einen dichten Raum (7) abgrenzt, der von einem Gasstrom durchspült wird und dessen Boden (2) aus transparentem Material zur Aufnahme einer mit dem Gasstrom in Kontakt stehenden Flüssigkeitsschicht (9) ausgelegt ist, welche Zelle (1) einerseits einen Ein-/Ausgang (10, 10') für die Erzeugung des Gasstroms zum Durchspülen des Raumes (7) aufweist, wobei der Eingang (10) mit der Sonde (30, 12) zur Entnahme atmosphärischer Luft verbunden ist, und andererseits einen Ein-/Ausgang (8, 8') für die Erzeugung der Flüssigkeitsschicht (9) auf dem Boden (2) aufweist, wobei der Eingang (8) mit der Quelle (15) zur Zellzüchtung (20) verbunden ist, damit letztere die Flüssigkeitsschicht bildet;
- Mittel (42, 45) zum Spülen des Bodens (2) der Expositionszelle;
- und ein System zur Inspektion des Bodens (2) der Expositionszelle, umfassend eine Vergrösserungsoptik (4) verbunden mit einer Kamera (3), Mittel zur Erfassung der von der Kamera erzeugten Signale von Quellenbildern, Mittel (46, 47, 48, 39) zur Verarbeitung der Signale, um verarbeitete Bilder zu erhalten, die Verhaltensänderungen der Immunabwehrzellen aufzuzeigen vermögen, sowie Mittel (5) zur Anzeige der verarbeiteten Bilder.

2. Biosensor nach Anspruch 1, **dadurch gekennzeichnet, dass** die Quelle (15) eine Kultur alveolärer Monozyten und Makrophagen des menschlichen Respirationstraktes enthält.

3. Biosensor nach Anspruch 1, **dadurch gekennzeichnet, dass** er zusätzlich eine Quelle chemoattraktiver Stoffe (33) für die Immunzellen sowie einen mit dieser Quelle verbundenen Eingang (13) in die Expositionszelle (1) für die Zuführung der chemoattraktiven Stoffe aufweist und dass die Mittel (46, 47) zur Verarbeitung der von der Kamera (3) gelieferten Quellenbilder Mittel sind, die Änderungen der Beweglichkeit der Abwehrzellen in Richtung der Zuführungsstelle der chemoattraktiven Stoffe auf dem Boden (2) unter dem Einfluss der zu analysierenden gasförmigen Luftprobe aufzuzeigen vermögen.

4. Biosensor nach Anspruch 1, **dadurch gekennzeichnet, dass** er Mittel (35) zur Beleuchtung des Bodens (2) der Beobachtungszelle (1) aufweist.

5. Biosensor nach Anspruch 1, **dadurch gekennzeichnet, dass** die Mittel zum Spülen des Bodens (2) der Expositionszelle (1) mit dem Eingang (8) zur Flüssigkeitsschicht (9) verbunden sind.
